# FASCICULE DE BREVET EUROPEEN

(11) **EP 0 680 968 B1**
(45) Date de publication et mention de la délivrance du brevet: **16.02.2000**
(21) Numéro de dépôt: 95400876.9
(22) Date de dépôt: 20.04.1995
(51) Int. Cl.: C07H 19/01, C07D 493/22

(54) **Procédé de préparation du ginkgolide B à partir du ginkgolide C**
Verfahren zur Herstellung von Ginkgolide B aus Ginkgolide C
Method for the preparation of ginkgolide B from ginkgolide C

(30) Priorité: 22.04.1994 GB 9408044
(43) Date de publication de la demande: 08.11.1995
(73) Titulaire: SOCIETE DE CONSEILS DE RECHERCHES ET D'APPLICATIONS SCIENTIFIQUES (S.C.R.A.S.), 75016 Paris (FR)
(72) Inventeur: Cazaux, Jean-Bernard, F-30390 Aramon (FR); Dafniet, Michel, F-13570 Barbentane (FR); Rebollo, Jacques, F-30210 Sernhac (FR); Teng, Beng-Poon, F-30400 Villeneuve-lez-Avignon (FR)
(74) Mandataire: Bourgouin, André

(56) Documents cités:
- GB-A- 2 254 612
- LIEBIGS ANN. CHEM. (1991), (1), 81-3, 1991 WEINGES, KLAUS ET AL 'Herstellung von Ginkgolid B aus Ginkgolid C'
- LIEBIGS ANN. CHEM. (1993), (9), 1023-7, 1993 WEINGES, KLAUS ET AL 'Herstellung von 1,10-Dihydroxy- und 1,7,10-Trihydroxyginkgolid aus 1,3,7,10-tetrahydroxyginkgolid'
- CARBOHYDRATE RESEARCH, vol. 110, 1982 AMSTERDAM NL, pages 19-41, BAER H.H. AND HANNA H.R. 'Desulfonyloxylations of some secondary p-toluenesulfonates of glycosides by lithium triethylborohydride; A high-yielding route to 2- and 3-deoxy sugars'

## Description

L'invention concerne des ginkgolides et, en particulier, un procédé pour convertir le ginkgolide C en ginkgolide B.

Les ginkgolides B et C sont des lactones diterpéniques, isolées à partir de feuilles et de racines d'arbres du ginko biloba. Les ginkgolides B et C sont les ginkgolides prédominants extraits ; ils sont extraits dans des proportions généralement comparables. Le ginkgolide B a été découvert comme étant un inhibiteur puissant du PAF-acéther (cf, par exemple, le brevet US 4 734 280). Le ginkgolide C a quelque activité, mais n'est pas aussi actif que le ginkgolide B. L'extraction des ginkgolides est un procédé coûteux. Par ailleurs, il n'existe pas de façon d'extraire sélectivement le ginkgolide B seul ; tous les ginkgolides sont extraits et ensuite isolés les uns des autres. Par conséquent, un procédé pour la conversion du ginkgolide C en ginkgolide B est d'un intérêt certain.

Les ginkgolides B et C ont quasiment la même structure chimique. La formule chimique du ginkgolide B est la suivante :

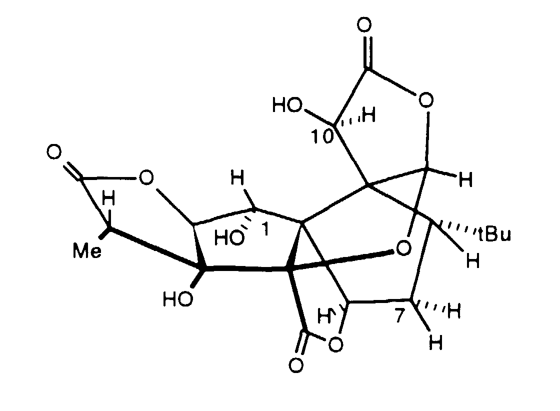

La formule chimique du ginkgolide C est la suivante :

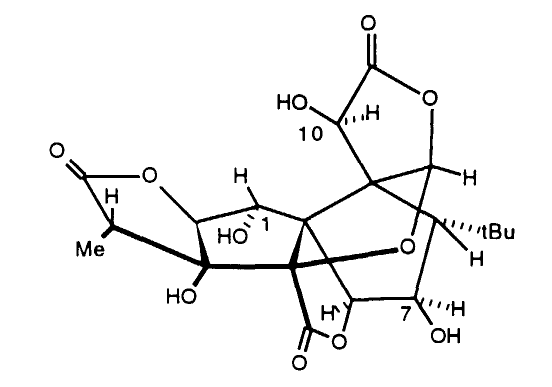

Comme on peut le constater, la seule différence entre ces 2 composés est que l'un des hydrogènes en position 7 du ginkgolide B est un groupe hydroxy dans le ginkgolide C.

Le brevet US 5 241 084 décrit un procédé pour convertir le ginkgolide C en B : le procédé, en 4 quatre étapes, comprend les étapes successives suivantes :
- protection du groupe hydroxy en position 10 du ginkgolide C par conversion en un alkyl ester, la réaction s'effectuant dans de la diméthylformamide à une température comprise entre 15 et 50° C pendant 4 à 10 heures ;
- activation du groupe hydroxy en position 7 du ginkgolide C résultant protégé en position 10, par conversion en un (R)thiocarbonylester, l'activation s'effectuant dans des conditions basiques à une température comprise entre 0 et 40°C pendant 1 à 24 heures ;
- désoxygénation du groupe activé en position 7 du ginkgolide C résultant activé en position 7 et protégé en position 10, par traitement avec de l'hydrure de tributyl étain ou du tris-(triméthylsilyl)silane, dans un solvant aprotique, en présence d'un générateur de radicaux libres, la réaction s'effectuant à une température comprise entre 70 et 110°C pendant 15 minutes à 3 heures sous atmosphère inerte ; et
- clivage du groupe protecteur du groupe hydroxy en position 10 du ginkgolide B protégé en position 10.

Bien que le procédé du brevet US 5 241 084 soit précieux dans la mesure où la conversion du ginkgolide B en ginkgolide C par un procédé en 4 étapes est beaucoup moins onéreux que l'extraction du ginkgolide B à partir de l'arbre ginkgo, un procédé encore plus simple est toujours plus souhaitable.

La désoxygènation de groupes hydroxy par la formation de sulfonates intermédiaires suivie de la réaction avec des borohydrures, a été mise en oeuvre pour des sucres (Carbohydrate Research, vol. 110, 1982, p. 19-41). Mais si, dans le cadre des sucres, la désoxygènation a nécessité au préalable la protection des autres groupes hydroxy, aucune protection n'a été nécessaire dans le cadre de la présente invention.

La présente invention concerne donc un procédé de conversion du ginkgolide C en ginkgolide B en seulement 2 étapes ; ce procédé comprend les 2 étapes successives suivantes :
- faire réagir le ginkgolide C avec un anhydride sulfonique pour obtenir un C-7 sulfonate de gingkolide C ; et
- faire réagir le C-7 sulfonate de ginkgolide C avec un borohydrure, la réaction éliminant le radical C-7 du sulfonate de ginkgolide C pour obtenir le ginkgolide B.

La première étape de ce procédé en 2 étapes peut-être réalisée en milieu basique à une température comprise entre environ -20°C et environ 35°C et pendant une période d'environ 15 minutes à environ 3 heures. Le solvant utilisé est de préférence la pyridine, un mélange de pyridine et de dichlorométhane, un mélange d'acitonitrile et triéthylamine, la 4-diméthylaminopyridine (DMAP) ou l'imidazole. La réaction s'effectue très préférentiellement dans de la pyridine en utilisant 1 à 4 équivalents d'anhydride trifluorométhanesulfonique.

Les anhydrides sulfoniques utilisables dans la présente invention peuvent être définis comme des anhydrides R-sulfonique dans lesquels R peut être un halogène, un alkyl inférieur en C₁-C₆, un alkyl inférieur substitué par un halogène, un phényl ou un phényl substitué. Les substituants préférés pour R sont méthyl, n-butyl, trifluorométhyl, toluène, p-nitrophényl, p-bromophényl et 2,4,6-trinitrophényl.

La seconde étape du procédé de la présente invention peut être réalisée dans un solvant aprotique, à une température entre environ 10°C et environ 30°C pendant une période d'environ 15 minutes à environ 3 heures.

Les borohydrures utilisables dans cette seconde étape peuvent être aussi définis comme des R-borohydrures dans lesquels R peut être un alcalin ou un ammonium. Le borohydrure de métal alcalin préféré est le borohydrure de sodium. Les borohydrures d'ammonium souhaitables sont les borohydrures de tétraarylalkylammonium et de tétraalkylammonium dans lesquels le groupe alkyl peut avoir de 1 à 6 atomes de carbone et le groupe aryl peut être un cycle simple ou double. Le borohydrure d'ammonium préféré est le borohydure de tétrabutylammonium. La seconde étape est de préférence réalisée dans du tétrahydrofurane (THF).

Ces aspects et d'autres aspects de la présente invention seront mieux compris en référence aux exemples suivants.

### EXEMPLE 1 :

Cet exemple illustre le procédé en 2 étapes pour la conversion du ginkgolide C en B.

### Etape 1

A une solution de pyridine (6 ml) dans du dichlorométhane (150 ml) refroidie à -15°C, on ajoute 11,4 ml d'anhydride trifluorométhanesulfonique à une température inférieure à -10°C. Une solution de ginkgolide C (26,4 g) dans de la pyridine (225 ml) est ajoutée à la même température. Le mélange est agité pendant 2 heures à -15°C puis on laisse la température remonter jusqu'à la température ambiante. Le mélange est concentré sous pression réduite et le résidu traité avec de l'acétate d'éthyl (150 ml). La solution obtenue est lavée avec de l'acide chlorhydrique 1N (75 ml) puis deux fois avec une solution de chlorure de sodium (75 ml). La phase organique est traitée avec du charbon actif (3 %), séché sur sulfate de magnésium et concentré sous vide. Le résidu résultant est traité avec un mélange de méthyl-t-butyl éther et d'heptane (250/300 ml). La suspension est filtrée et le solide lavé deux fois à l'heptane (50 ml). On obtient le C-7 trifluorométhane sulfonate de ginkgolide C (rendement 91 %).

### Etape 2

Une solution de borohydrure de tétrabutylammonium (11,47 mg) dans du THF (50 ml) est ajoutée goutte à goutte à la solution de C-7 trifluorométhane sulfonate de ginkgolide C (25,5 g) dans 200 ml de THF, à 20°C. Le mélange réactionnel est agité pendant une heure à 20°C puis refroidi à 10°C et traité avec du méthanol (25 ml). La solution est concentrée et le résidu traité avec de l'acétate d'éthyl (150 ml). Le mélange ainsi obtenu est lavé avec de l'hydroxide d'ammonium (60 ml), une solution de 20% en poids de chlorure de sodium (60 ml) et de l'acide chlorhyrique 1N (60 ml) puis de nouveau une solution de chlorure de sodium (15 ml). Le solvant est éliminé sous pression réduite et le résidu obtenu traité avec un mélange eau/éthanol (300/100 ml). La solution résultante est refroidie et le produit recrystallise. Le produit est filtré, lavé puis séché. Le produit résultant est le ginkgolide B (rendement 85,8 %).

### EXEMPLE 2 :

L'exemple 1 est répété à l'exception, dans l'étape 1, de l'anhydride trifluorométhanesulfonique qui est remplacé par l'anhydride méthanesulfonique ; le rendement de la première étape est de 90,5 %. La seconde étape est effectuée de la même manière que celle de l'exemple 1 (rendement 84 %).

### EXEMPLE 3 :

L'exemple 1 est répété à l'exception, dans l'étape 1, de l'anhydride trifluorométhanesulfonique qui est remplacé par l'anhydride n-butane sulfonique ; le rendement de la première étape est de 87 %. Et dans la seconde étape, le borohydrure de tétrabutylammonium est substitué par le borohydrure de sodium (rendement 80,2%).

### EXEMPLE 4 :

L'exemple 2 est répété à l'exception, dans l'étape 1, de l'anhydride trifluorométhane sulfonique qui est remplacé par l'anhydride benzènesulfonique ; le rendement de la première étape est de 86 %. La seconde étape est effectuée de la même manière que celle de l'exemple 1 (rendement 83 %).

### EXEMPLE 5 :

L'exemple 1 est répété à l'exception, dans l'étape 1, de l'anhydride trifluorométhanesulfonique qui est remplacé par l'anhydride toluènesulfonique ; le rendement de la première étape est de 90 %. La seconde étape est effectuée de la même manière que celle de l'exemple 1 (rendement 86,7 %).

### EXEMPLE 6 :

L'exemple 1 est répété à l'exception, dans l'étape 1, l'anhydride trifluorométhanesulfonique qui est remplacé par l'anhydride 2,4,6-trinitrobenzènesulfonique ; le rendement de la première étape est de 86 %. La seconde étape est effectuée de la même manière que celle de l'exemple 1 (rendement 83 %).

## Revendications

1. Procédé de préparation du ginkgolide B à partir du ginkgolide C, le procédé comprenant les 2 étapes successives suivantes :
(a) faire réagir le ginkgolide C avec un anhydride sulfonique pour obtenir un C-7 sulfonate de gingkolide C, et
(b) faire réagir le C-7 sulfonate de ginkgolide C avec un borohydrure, la réaction éliminant le radical C-7 du sulfonate de ginkgolide C pour obtenir le ginkgolide B.

2. Procédé selon la revendication 1, dans lequel la réaction de l'étape (a) est effectuée en milieu basique à une température comprise entre environ -20°C et environ 35°C et pendant une période d'environ 15 minutes à environ 3 heures.

3. Procédé selon l'une des revendications 1 à 2, dans lequel la réaction de l'étape (b) est effectuée dans un solvant aprotique, à une température entre environ 10°C et environ 30°C pendant une période d'environ 15 minutes à environ 3 heures.

4. Procédé selon l'une des revendications 1 à 3, dans lequel l'anhydride sulfonique est un anhydride R-sulfonique dans lequel R est sélectionné parmi le groupe comprenant un halogène, un alkyl inférieur en C₁-C₆, un alkyl inférieur substitué par un halogène, un phényl, et un phényl substitué.

5. Procédé selon la revendication 4, dans lequel R est sélectionné parmi le groupe comprenant méthyl, n-butyl, trifluorométhyl, toluène, p-nitrophényl, p-bromophényl et 2,4,6-trinitrophényl.

6. Procédé selon l'une des revendications 1 à 6, dans lequel le borohydrure est un R-borohydrure dans lesquel R est sélectionné parmi le groupe comprenant les métaux alcalins et les ammoniums.

7. Procédé selon la revendication 6, dans lequel le métal alcalin est le sodium.

8. Procédé selon la revendication 6, dans lequel l'ammonium est un tétraarylalkylammonium ou un tétraalkylammonium.

9. Procédé selon la revendication 8, le borohydrure est le borohydure de tétrabutylammonium.

10. Procédé selon l'une des revendications 1 à 9, dans lequel la réaction de l'étape (a) est effectuée dans un solvant sélectionné parmi le groupe comprenant la pyridine, un mélange de pyridine et de dichlorométhane, un mélange d'acitonitrile et triéthylamine, la 4-diméthylaminopyridine et l'imidazole.

11. Procédé selon la revendication 10, dans lequel la réaction de l'étape (a) est effectuée dans de la pyridine.

12. Procédé selon l'une des revendications 1 à 11, dans lequel la réaction de l'étape (b ) est effectuée dans du tétrahydrofurane.

## Patentansprüche

1. Verfahren zur Herstellung von Ginkgolid B aus Ginkgolid C, wobei das Verfahren die folgenden zwei aufeinanderfolgenden Stufen umfaßt:
(a) Reagieren von Ginkgolid C mit einem Sulfonanhydrid, um ein C-7 Sulfonat von Ginkgolid C zu erhalten; und
(b) Reagieren des C-7 Sulfonats von Ginkgolid C mit einem Borwasserstoff, wobei die Reaktion das C-7 Radikal des Sulfonats von Ginkgolid C beseitigt, um Ginkgolid B zu erhalten.

2. Verfahren nach Anspruch 1, bei dem die Reaktion von Stufe (a) in einem basischen Medium bei einer Temperatur zwischen etwa -20°C und etwa 35°C über einen Zeitraum von etwa 15 Minuten bis etwa 3 Stunden stattfindet.

3. Verfahren nach einem der Ansprüche 1 bis 2, bei dem die Reaktion von Stufe (b) in einem aprotischen Lösungsmittel bei einer Temperatur zwischen etwa 10°C und etwa 30°C über einen Zeitraum von etwa 15 Minuten bis etwa 3 Stunden stattfindet.

4. Verfahren nach einem der Ansprüche 1 bis 3, bei dem das Sulfonanhydrid ein R-Sulfonanhydrid ist, wobei R ausgewählt wird aus der Gruppe bestehend aus einem Halogen, einem niederen C₁-C₆ Alkyl, einem durch ein Halogen substituierten niederen Alkyl, einem Phenyl und einem substituierten Phenyl.

5. Verfahren nach Anspruch 4, bei dem R ausgewählt wird aus der Gruppe bestehend aus Methyl, n-Butyl, Trifluormethyl, Toluol, p-Nitrophenyl, p-Bromphenyl und 2,4,6-Trinitrophenyl.

6. Verfahren nach einem der Ansprüche 1 bis 6, bei dem der Borwasserstoff ein R-Borwasserstoff ist, wobei R ausgewählt wird aus der Gruppe bestehend aus Alkalimetallen und Ammonium.

7. Verfahren nach Anspruch 6, bei dem das Alkalimetall Natrium ist.

8. Verfahren nach Anspruch 6, bei dem das Ammonium ein Tetraarylalkylammonium oder ein Tetraalkylammonium ist.

9. Verfahren nach Anspruch 8, bei dem der Borwasserstoff Tetrabutylammoniumborwasserstoff ist.

10. Verfahren nach einem der Ansprüche 1 bis 9, bei dem die Reaktion von Stufe (a) in einem Lösungsmittel stattfindet, ausgewählt aus der Gruppe bestehend aus Pyridin, einem Gemisch aus Pyridin und Dichlormethan, einem Gemisch aus Acetonitril und Triethylamin, 4-Dimethylaminopyridin und Imidazol.

11. Verfahren nach Anspruch 10, bei dem die Reaktion von Stufe (a) in Pyridin stattfindet.

12. Verfahren nach einem der Ansprüche 1 bis 11, bei dem die Reaktion von Stufe (b) in Tetrahydrofuran stattfindet.

## Claims

1. Process for the preparation of ginkgolide B from ginkgolide C, the process comprising the following 2 successive stages:
(a) reacting ginkgolide C with a sulphonic anhydride in order to obtain a C-7 sulphonate of gingkolide C, and
(b) reacting the C-7 sulphonate of ginkgolide C with a borohydride, the reaction eliminating the C-7 radical of the sulphonate of ginkgolide C in order to obtain ginkgolide B.

2. Process according to claim 1, in which the reaction of Stage (a) is carried out in a basic medium at a temperature comprised between approximately -20°C and approximately 35°C and for a period of approximately 15 minutes to approximately 3 hours.

3. Process according to one of claims 1 to 2, in which the reaction of Stage (b) is carried out in an aprotic solvent, at a temperature between approximately 10°C and approximately 30°C for a period of approximately 15 minutes to approximately 3 hours.

4. Process according to one of claims 1 to 3, in which the sulphonic anhydride is an R-sulphonic anhydride in which R is selected from the group comprising a halogen, a lower C₁-C₆alkyl, a lower alkyl substituted by a halogen, a phenyl, and a substituted phenyl.

5. Process according to claim 4, in which R is selected from the group comprising methyl, n-butyl, trifluoromethyl, toluene, p-nitrophenyl, p-bromophenyl and 2,4,6-trinitrophenyl.

6. Process according to one of claims 1 to 6, in which the borohydride is an R-borohydride in which R is selected from the group comprising the alkali metals and the ammoniums.

7. Process according to claim 6, in which the alkali metal is sodium.

8. Process according to claim 6, in which the ammonium is a tetraarylalkylammonium or a tetraalkylammonium.

9. Process according to claim 8, the borohydride is tetrabutylammonium borohydride.

10. Process according to one of claims 1 to 9, in which the reaction of Stage (a) is carried out in a solvent selected from the group comprising pyridine, a mixture of pyridine and dichloromethane, a mixture of acetonitrile and triethylamine, 4-dimethylaminopyridine and imidazole.

11. Process according to claim 10, in which the reaction of Stage (a) is carried out in pyridine.

12. Process according to one of claims 1 to 11, in which the reaction of Stage (b ) is carried out in tetrahydrofuran.
